# EUROPÄISCHE PATENTSCHRIFT

(11) **EP 0 367 161 B1**
(45) Veröffentlichungstag und Bekanntmachung des Hinweises auf die Patenterteilung: **19.06.1996**
(21) Anmeldenummer: 89120054.5
(22) Anmeldetag: 28.10.1989
(51) Int. Cl.: C07K 1/12, C12P 21/02, C07K 14/62

(54) **Verfahren zur selektiven Spaltung von Fusionsproteinen**
Process for selectively hydrolising fusion proteins
Procédé pour l'hydrolyse sélective d'une protéine de fusion

(30) Priorität: 03.11.1988 DE 3837271
(43) Veröffentlichungstag der Anmeldung: 09.05.1990
(73) Patentinhaber: HOECHST AKTIENGESELLSCHAFT, D-65926 Frankfurt am Main (DE)
(72) Erfinder: Vértesy, Lászlo, Dr., D-6239 Eppstein/Taunus (DE); Sauber, Klaus, Dr., D-6232 Bad Soden am Taunus (DE); Ehlers, Eberhard, Dr., D-6238 Hofheim am Taunus (DE)

(56) Entgegenhaltungen:
- EP-A- 0 161 629
- EP-A- 0 195 691
- EP-A- 0 281 090
- EP-A- 0 281 090
- BIOTECHNOLOGY, Band 7, 1989, Riverton, US; K.-P. KOLLER et al., Seiten 1055-1059

## Beschreibung

Die Erfindung betrifft ein Verfahren zur Herstellung von Polypeptiden oder Proteinen durch enzymatische Spaltung eines Fusionsproteins mittels einer Trypsin-ähnlichen Endopeptidase.

Die zunehmende Bedeutung der rekombinanten DNA-Technologie zur Gewinnung von Polypeptiden und Proteinen erfordert die Entwicklung neuer Verfahren für die Anreicherung und Reinigung der Produkte, die den (neuen) Ausgangsmaterialien angepaßt sind.

Die Expression von Proinsulinderivaten mit verkürzter C-Kette in Hefezellen wird in EP-A-0 195 691 beschrieben. Die Verwendung des Tendamistatgens zur Herstellung von Fusionsproteinen in Streptomyceten wird in EP-A-0 281 090 beschrieben.

Bei der semisynthetischen Herstellung von Humaninsulin, das zur Behandlung des Diabetes mellitus eingesetzt wird, nach dem sogenannten "Zweistufen-Verfahren" erfolgt der Austausch des Aminosäureesters in der B³⁰ Position (Schweineinsulin = Ala → Humaninsulin = Thr) in zwei aufeinanderfolgenden getrennten Reaktionsstufen. In der ersten Reaktionsstufe wird der B³⁰-Ala-Rest des Schweineinsulins mit trypsinähnlichen Proteasen enzymatisch abgespalten und in der zweiten Reaktionsstufe wird dann ein Threoninderivat - z. B. Threonin-tert.-butylester (ThrOBu^{t}) - angekuppelt, was ebenfalls in Gegenwart von trypsinähnlichen Enzymen geschieht. Als Enzym für die beiden Reaktionsstufen wird z. B. von K. Morihara et al., Biochemical and Biophysical Research Communications Vol. 92, No. 2, 1980, S. 396 - 402, Achromobacter Protease I beschrieben. Es handelt sich um eine sogenannte Endopeptidase, die die Peptidketten speziell an der Carboxylseite der Aminosäure Lysin spaltet und gegebenenfalls auch wieder aufbaut.

Nach beendeter Alanin-Abspaltung und Threoninderivat-Ankupplung sind die mit dem Threoninderivat eingeführten Schutzgruppen zwecks Erhalts des schutzgruppenfreien Humaninsulins wieder abzuspalten; dafür sind verschiedene Methoden bekannt, wovon gegebenenfalls die Schutzgruppenabspaltung mittels CF₃COOH bevorzugt ist.

Die Abspaltung des B³⁰-Ala-Rests aus Schweineinsulin und die Ankupplung eines Threoninderivats kann auch in einem einzigen Reaktionsschritt ("Einstufen-Verfahren) erfolgen. Auch hier muß in Gegenwart eines Enzyms gearbeitet werden, wobei als Enzyme praktisch ausschließlich Trypsin und Trypsin-ähnliche Endopeptidasen in Frage kommen; vgl. z.B. EP-B-56 951. Nach beendeter "Transpeptidierung" sind die mit dem Threoninderivat eingeführten Schutzgruppen wieder abzuspalten.

Man hat aus naheliegenden Gründen auch versucht, bei der Herstellung von Humaninsulin von dem natürlichen Ausgangsmaterial Schweineinsulin wegzukommen, was seit der Entwicklung der Gentechnologie auch - zumindest teilweise - gelungen ist. So wird z. B. in EP-B-89 007 ein Verfahren zur Herstellung von Insulinen - insbesondere von Humaninsulin - aus gentechnologisch zugänglichen Präproinsulin-Analogen beschrieben, bei dem man Präproinsulin-Analoga bei einem pH-Wert unterhalb des isoelektrischen Punkts der zugehörigen Insuline mit Hilfe von Trypsin oder einer Trypsin-ähnlichen Endopeptidase mit einem Ester natürlicher Aminosäuren oder solchen Derivaten daraus, die Schutzgruppen enthalten, umsetzt (Transpeptidierung) und danach die Estergruppe und die gegebenenfalls vorhandenen Schutzgruppen abspaltet. Für die Herstellung von Humaninsulin wird als Aminosäurederivat bei dieser Reaktion vorzugsweise Thr(Bu^{t})-OBu^{t} eingesetzt.

Als Ausgangs-Präproinsulin-Analoga sind in der genannten EP-B solche angegeben, die am N-Terminus des Proinsulins Lysin oder Arginin oder deren Acylaminoacyl-Reste als Carboxylende einer Prä-Sequenz tragen; nach der EP-B ist die Formel dieser Präproinsulin-Analoga: 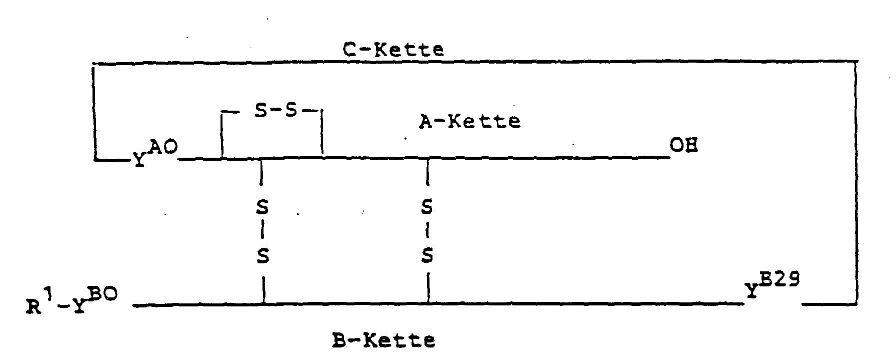 in der Y Lys oder Arg und R¹ Wasserstoff, eine L-Aminosäure oder den nachfolgend beschriebenen Peptidrest X bedeuten. X kann jedes als Signalsequenz dienendes Peptid sein. Als Proinsulin-Teil eignet sich jedes Produkt, das mit einem natürlichen oder synthetisch nach bekannten Nukleotid-Darstellungsverfahren variierten Proinsulinen codiert wird. Analoge Präproinsuline mit verkürztem C-Peptid-Segment, wie es z.B. beim C-Peptid von Rind gegenüber dem C-Peptid vom Schwein der Fall ist, kommen ebenfalls für das Verfahren in Frage. Ebenso sind Human-Präproinsulin-Analoga geeignet. Wesentlich für die Struktur des C-Peptids ist, daß es über eine basische L-Aminosäure Y = L-Arg oder L-Lys mit dem Glycin der Insulin-A-Kette verknüpft ist; d.h., daß eine Struktur Y-(AS)ₙ-Y vorliegt, wobei AS alle codierbaren Aminosäuren bedeutet und n = 0-35 ist. Aminosäureester und/oder deren Derivate mit freier Aminogruppe können für das Verfahren auch so gewählt werden, daß gewünschtenfalls ein Insulinanalogon von nicht natürlicher Sequenz entsteht.

Bei der enzymatischen Reaktion wird der Prä-Teil R¹-Y^{B0} sowie die C-Kette Y^{A0}-B³⁰ abgespalten und an Position B³⁰ das entsprechende Aminosäurederivat angekuppelt.

Es ist vorstellbar, daß man ohne Zusatz des entsprechenden Aminosäurederivats hier auch Des-B³⁰-Insuline erhalten kann.

Obwohl dieses Verfahren insbesondere wegen seiner Unabhängigkeit von tierischen Insulinen als Ausgangsmaterialien erhebliche Vorteile besitzt, ist doch die Notwendigkeit der säulenchromatographischen Reinigung der Reaktionsprodukte und der dabei auftretende - oft nicht unerhebliche - Substanzverlust ein gewisser Nachteil. Die Notwendigkeit der säulencromatographischen Reinigung besteht deswegen, weil bei der enzymatischen Reaktion häufig Peptid-Spaltprodukte entstehen, die sich von dem gewünschten Insulin (-Derivat) vor allem wegen einer ähnlichen Molekülkettenlänge nur schwierig trennen lassen.

Es bestand nun die Aufgabe, das Verfahren der vorerwähnten EP-B-89 007 so abzuwandeln, daß man Polypeptide oder Proteine - vorzugsweise Des-B³⁰-Insuline oder Humaninsulin - enthält und daß die gewünschten Produkte in einer leichter reinigbaren Form mit weniger Verlusten bei der Reinigung anfallen.

Diese Aufgabe konnte erfindungsgemäß durch enzymatische Spaltung von spezielle Fusionsproteinen mit einer Trypsin-ähnlichen Endopeptidase gelöst werden.

Erfindungsgegenstand ist daher ein Verfahren zur Herstellung von B³⁰-Desthreonin-Humaninsulin durch enzymatische Spaltung eines Fusionsproteins mit einer trypsin-ähnlichen Endopeptidase, dadurch gekennzeichnet, daß man ein Fusionsprotein verwendet, das durch Expression eines Strukturgens in einer Streptomycetenzelle erhalten wird, wobei das Strukturgen aus der Tendamistatsequenz besteht, auf das die Gensequenz für das Brückenglied mit den Aminosäuren Phe Asn Ala Met Ala Thr Gly Asn Ser Asn Gly Lys folgt und hierauf das Gen für ein menschliches Proinsulinderivat folgt, bei dem die B-Kette über ein Brückenglied an die A-Kette gebunden ist, das für Lys oder Lys-Lys codiert.

Die bei dieser enzymatischen Reaktion entstehenden Des-B³⁰-Insuline sind aufgrund des Tendamistat-Restes des Fusionsproteins besonders leicht zu reinigen. Die Verbindungen sind in wäßrigem Medium leicht löslich, leicht kristallisierbar und haben eine Affinität zu α-Amylasen, was wiederum zu ihrer Abtrennung genutzt werden kann. Beispielsweise sind die Des-B³⁰-Insuline mit geringerem Aufwand und geringeren Verlusten reinigbar als die Reaktionsprodukte des Verfahrens der EP-B-89 007, In günstigen Fällen kann die Aufarbeitung des Reaktionsansatzes sogar lediglich durch Kristallisation - also ohne die Notwendigkeit einer säulenchromatographischen Reinigung - erfolgen. Es ist überraschend, daß die Tendamistatsequenz bei der erfindungsgemäßen Reaktion durch die Trypsin-ähnlichen Endopeptidasen nicht gespalten wird, weil diese Tendamistatsequenz einen Lysylrest besitzen, an welchem - beispielsweise im Gegensatz zu den Lysylresten in den Positionen A⁰, B⁰ und B²⁹ der Ausgangsverbindungen der Des-B³⁰-Insuline (siehe Seite 3) - keine Spaltung erfolgt; durch eine solche Spaltung würden Bruchstücke entstehen, die sich von den Des-B³⁰-Insulinen nur mit u.U. erheblichem Aufwand abtrennen lassen.

Als Tendamistatsequenz kommen praktisch alle Reste der literaturbekannten α-Amylase-Inhibitoren in Frage, wie sie z. B. beschrieben sind in: L. Vértesy et al., Euro. J. Biochem. 141, 505-512 (1984); L. Vértesy, D. Tripier, FEBS-Letters Vol. 185, 187-190 (1985); 0. Hoffmann et al., Biol. Chem. Hoppe-Seylers Vol. 366, 1161-1168 (1985); H. Murai et al., J. Biochem. 97, 1129-33 (1985); S. Murao et al., Agric. Biol. Chem. 49, 107-110 (1985) und 49, 793-797 (1985); Japan Kokai 75/77594 (20. Nov. 1973) H. Goto et al.

Bevorzugte Tendamistatsequenz ist:

-Leu-Cys-S-S-Cys-Leu-Gly-Glu-Thr-Asp-Asp-Glu-Tyr-Val-Val-Lys
Arg
Ala Tyr
Leu Ala
Tyr Val
Arg Ala
Ala Pro
His Gly
Gly Gln
His Ile
Ser Thr
Gly Thr
Ile Val
Tyr Gly
Gly Asp
Val-Thr-Val-Thr-Glu-Ala-Cys-S-S-Cys-Ser-Pro-Ala-Pro-Glu-Ser-Val-Thr-Thr-Asp
Gly NH2
Asn Val
Asp Thr
Ala Leu
Gln Tyr
Ser Gln
Tyr Ser
Arg Trp

Die Herstellung der Fusionsproteine erfolgt im Mikroorganismus nach allgemein bekannten Methoden durch Voransetzen einer Sequenz eines Fremdproteins (α-Amylase-lnhibitor) vor die Aminosäuresequenz des gewünschten Polypeptids oder Proteins (siehe z.B. F.A.O. Harston, Biochem. J. 240 (1986) 1-12). Die bevorzugten Präproinsulin-Analoga werden gentechnologisch, vorzugsweise nach dem Verfahren der gleichzeitig eingereichten Patentanmeldung "Verfahren zur Herstellung eines Insulin-Vorprodukts in Streptomyceten" (EP 0 367 163) hergestellt. Die Isolierung der, in der Regel ins Kulturmedium ausgeschiedenen Insulinvorprodukte erfolgt vorzugsweise aus den Filtraten der Fermentationsflüssigkeitn. Es wurde gefunden, daß viele Eigenschaften der genannten Präproinsulin-Analoga dem Verhalten der genannten α-Amylaseinhibitoren ähneln. Somit können viele Isolierungs- und Reinigungsschritte der aufgeführten mikrobiellen Amylaseinhibitoren für die Gewinnung der Fusionsproteinvorprodukte insbesondere der Insulinvorprodukte herangezogen werden.

Solche Reinigungsschritte sind Fällungen mit Salzen wie Kochsalz, Ammoniumsulfat u.a., mit Säuren wie z.B. Metaphosphorsäure, Tannin, Trichloressigsäure, Schwefelsäure und ähnlichen mit Schwermetallsalzen und anderen Fällungsmitteln wie z.B. Polyethyleniminen, Bentonit oder anderen.

Während gentechnologisch gewonnene Proteine häufig unerwünschte Löslichkeitsverhalten zeigen, und damit sich einer leichten Bearbeitbarkeit entziehen, können die klar löslichen Präproinsulinanaloga, z.B. sehr elegant durch Ultrafiltration konzentriert und gleichzeitig entsalzt werden. Geeignet sind für die Ultrafiltration z.B. käufliche Cellulosemembranen.

Ebenso können Adsorptionsharze zur Anreicherung und Reinigung von Präproinsulinanaloga verwendet werden. Solche Adsorptionsharze auf Polystyrol- oder Styrol/Divinylbenzol-Copolymerisatbasis sind im Handel erhältlich und können unter der Bezeichnung ®Amberlite XAD (Rohm & Haas, USA), ®Diaion HP-20 (Mitsubishi Chemical Corp.) u.a., bezogen werden.

Das Isolieren eines Proteins mit Adsorptionsharzen wie z.B. Diaion HP-20, ist bereits in DOS 3038130 beschrieben. Gentechnologisch gewonnene Proteine wie z.B. Insulinvorprodukte konnten aber mit Adsorptionsharzen nicht oder nur unter großen Verlusten angereichert werden. Dies lag u.a. an der zu festen Haftung des Wertstoffes am Träger. Überraschenderweise wurde nun festgestellt, daß man Adsorptionsharze doch vorteilhaft einsetzen kann, wenn man für mildere, geringere Bindung des Materials sorgt. Dieses Inaktivieren kann z.B. durch Behandlung des Trägers und/oder durch geeignete Zusätze zu dem Trenngut geschehen. Als Beispiel für die Behandlung des Trägers seien genannt: Waschen mit wäßrigen organischen Lösungsmitteln (2-70 %, vorzugsweise 5-30 % Lösungsmittelanteile). Als organische Lösungsmittel kommen solche in Frage, die mit Wasser mischbar sind; bevorzugt niedere Alkohole und Aceton. Die Vorbehandlung des Trägers kann weiter durch Waschen mit wäßrigen Detergentienlösungen geschehen, beispielsweise mit 0,02-3 %iger ®Triton x-100-Lösung, vorzugsweise jedoch mit 0,1-1 % Lösung, geeignet sind aber natürlich auch eine große Zahl anderer Detergentien.

Ebenfalls geeignet ist das Versetzen der Trenngutlösung und/oder des Elutionsmittels mit geeigneten Zusätzen. Solche Zusätze sind niedere, mit Wasser mischbare organische Lösungsmittel und Detergentien jeweils in den vorhin genannten Konzentrationen oder sogenannten Chaotrope Substanzen wie z.B. Kaliumperchlorat (0,1-3 %), Harnstoff (1-8 molar) oder Guanidinium-hydrochlorid u.a.

Die gegebenenfalls angereicherten, entsalzten Präproinsulinanaloga können weiter durch Ionenaustauscherchromatographie an Kationenaustauscher als auch an Anionenaustauscher gereinigt werden. Geeignete Kationenaustauscher sind z.B. SP-®Sephadex, (Pharmacia, Schweden) CM-Cellulose, S-®Sepharose (Pharmacia, Schweden), ®Fractogel TSK CM (E. Merck, Darmstadt), ®Fractogel TSK SP (E. Merck, Darmstadt) und andere. Geeignete Anionenaustauscher sind ®Fractogel TSK DEAE (E. Merck, Darmstadt), DEAE-Cellulose, DEAE-®Sephadex (Pharmacia, Schweden), Q-Sepharose, QAE-Sephadex, QAE-Cellulose und weitere. Die Trennungen werden in an sich bekannter Weise in wäßriger Lösung durchgeführt. Es hat sich als vorteilhaft erwiesen, bei den Trennungen in manchen Fällen jedoch dem Wasser Lösungsvermittler und/oder Chaotrope Substanzen hinzuzufügen. Solche Zusätze sind 2-8 molare Harnstoffe, Betain, niedere Alkohole, wie Methanol, Isopropanol, Ethanol, Ethylenglykol und andere. Geeignete Reinigungsoperationen sind weiter die hydrophobe Chromatographie z.B. an Phenyl-Sepharose, Molekularsiebchromatographie z.B. mittels Biogel, präparative Hochdruckflüssigkeitschromatographie oder die Chromatographie an einer mit Antikörper gegen α-Amylase-Inhibitor beladenen Säule.

Die α-Amylase-Inhibitor-ähnlichen Eigenschaften des Insulinvorproduktes ermöglichen auch die Reinigung durch Kristallisation.

Kristalliert werden kann sowohl in der Nähe des jeweiligen isoelektrischen Punktes oder im sauren Milieu ggf. unter Verwendung von Zusätzen wie z.B. Kochsalz oder anderen. Möglich ist auch die Kristallisation aus wäßriger Lösung durch Zugabe von - die Kristallisation fördernden - Mitteln wie z.B. von Schwermetallionen wie Zn²⁺, Cu²⁺ und anderen der von Pikrinsäure und ähnlichen.

Es ist weiter sehr vorteilhaft, die α-Amylasehemmende Eigenschaft der Präproinsulinanaloga für die Reinigung auszunützen. Es ist nämlich gefunden worden, daß auch die gentechnologisch gewonnenen Proteine, die die Aminosäuresequenz der Inhibitoren tragen, die α-Amylase durch Komplexbildung hemmen. Die Inhibitor/α-Amylasekomplexe sind schwerer löslich als die Ausgangskomponenten und können daher zur Abtrennung herangezogen werden. Man kann auch mit auf Träger fixierter Amylase die Präproinsulinanaloga aus dem Kulturfiltrat selektiv abtrennen und anschließen den gefundenen Komplex mit geeigneten Mitteln, wie z.B. pH- oder Salzgradienten trennen. Vorteilhaft ist eine solche Verfahrensweise auch bei der Aufarbeitung des proteolytischen Hydrolysates der Präproinsulinanaloga. Aus dem Reaktionsansatz wird hier mit fixierter α-Amylase das nicht mehr benötigte Inhibitor-Spaltprodukt entfernt. Das verbleibende Des-B³⁰-Humaninsulin ist dann bereits so rein, daß es direkt der Kristallisation zugeführt werden kann. Die Kristallisation erfolgt nach an sich bekannten Methoden.

Für die enzymatische Spaltung der Präproinsulinanaloga werden gereinigte Vorprodukte eingesetzt, es können aber auch nur partial angereinigte Ausgangsmaterialien angewandt werden.

Die Konzentration der Präproinsulinanaloga im Reaktionsansatz kann in relativ weiten Grenzen schwanken. Bevorzugt ist eine Konzentration von etwa 0,02 - 15 Gew.-%, insbesondere von etwa 0,05 - 5 Gew.-%, bezogen auf den gesamten Reaktionsansatz.

Als Trypsin-ähnliche Endopeptidasen kommen die Endopeptidasen in Frage, die aus der Literatur als Trypsinähnlich bekannt sind, d, h. solche, die spezifisch Peptidbindungen am Carboxylende basischer Aminosäuren spalten; einige entsprechende Literaturstellen sind in der vorerwähnten EP-B 89 007 angegeben, oder solche, die selektiv hinter Lysin spalten, siehe z.B. EP-A-092 829 bzw. US-A-4,414,332. Bevorzugte Trypsin-ähnliche Endopeptidase ist Lysylendopeptidase aus Lysobacter enzymogenes, welche Peptidbindungen am Carboxylende speziell der basischen Aminosäure Lysin spaltet. Einschlägige Literatur betreffend Lysylendopeptidase ist z. B.
P. A. Jeckel et al., Analytical Biochemistry, Band 134 (1983), S. 347 - 354 und
T. Masaki et al., Biochim. Biophys. Akad., Band 660, Seiten 44f und 51f (1981).

Die Konzentration der Trypsin-ähnlichen Endopeptidase in der Reaktionsmischung wird zweckmäßig zwischen etwa ein Fünfzigstel und ein Zehntausendstel, vorzugsweise zwischen 0,5 und 1,5 % (w/w), insbesondere von etwa ein Tausendstel des Gewichts des Fusionsproteins der Formel I eingestellt.

Die enzymatische Spaltung der Präproinsulinanaloga wird in wäßriger Lösung, gegebenenfalls in Gegenwart eines Lösungsvermittlers (wie z. B. Harnstoff, Isopropanol etc,), bei pH zwischen etwa 5 und 11, vorzugsweise zwischen etwa 7 und 9,5, durchgeführt. Zur Einstellung und Konstanthaltung des pH-Werts ist es zweckmäßig, eines der üblichen Puffersysteme wie z. B. Phosphatpuffer, Tris/HCl, Na₂CO₃/NaHCO₃ zu verwenden.

Die Temperatur der enzymatischen Spaltung kann zwischen etwa 1 und 60°C liegen; vorzugsweise arbeitet man zwischen etwa 15 und 40°C.

Die Spaltung ist im allgemeinen in etwa 0,1 bis 2 Stunden beendet, doch können - je nach den speziellen Reaktionsbedingungen - auch kürzere oder längere Zeiten resultieren.

Nach Beendigung der enzymatischen Spaltung - was z. B. chromatographisch festgestellt werden kann - wird eine etwaige weitere - unerwünschte - Spaltung durch Veränderung des Reaktionsmilieus auf bekannte Weise gestoppt, z. B. durch pH-Veränderung, Abkühlung, Zusatz von Inhibitoren etc., und das gebildete Des-B³⁰-Insulin nach an sich bekannten Verfahren isoliert. Die Isolierung kann zwar wie bei den bekannten einschlägigen Verfahren durch Chromatographie erfolgen, doch ist im vorliegenden Fall im Prinzip auch die Isolierung durch Kristallisation möglich, weil das Des-B³⁰-Insulin sich von den Nebenprodukten bezüglich der Kettenlänge und damit des Molekulargewichts erheblich unterscheidet.

Eine "Transpeptidierung" wird in wäßriger-organischer Lösung (z.B. Wasser: DMF, Wasser BMSO) aller Mischungsverhältnisse, vorzugsweise Mischungen von 1:4 bis 1:1 (Lösungsmittel : Wasser) durchgeführt. Der pH-Wert sollte zwischen 4 und 8, vorzugsweise zwischen 5 und 6 liegen; die Temperatur zwischen 1 und 50 °C, vorzugsweise zwischen 15 und 30 °C.

Nach Beendigung der enzymatischen "Transpeptidierung", was z.B. durch HPLC festgestellt werden kann, wird eine mögliche Rückreaktion, wie z.B. zu Des-B³⁰-Insulin, durch Zugabe von Inhibitoren, wie z.B. Aprotinin, Butylamin oder Tosyl-L-Lysinchloromethyl-Keton verhindert.

Danach kann problemlos verdünnt und präparativ über Säulenchromatographie gereinigt, und somit auch von nicht umgesetzten Ausgangsmaterial abgetrennt werden. Ebenfalls möglich ist hier eine Kristallisation.

Die Abspaltung der Schutzgruppen erfolgt nach literaturbekannten Methoden.

Das erfindungsgemäße Verfahren soll nun durch die folgenden Beispiele näher erläutert werden.

### Beispiel 1

30 l Kulturbrühe, die zur Gewinnung von dem Konstrukt pGF2 fermentiert wurde, zentrifugiert man, anschließend wird nochmal sterilfiltriert. Die, auf pH 7,2 eingestellte Lösung versetzt man mit 3 l Isopropanol und trägt auf eine 4 l-®Diaion HP-20 Säule auf, die vorher mit 10 %igem Isopropanol gewaschen wurde. Durch Anlegen eines 10-50 %igem Isopropanolgradienten wird das neukonstruierte Protein pGF2 von der Säule eluiert. Die Wertproduktenthaltenden Fraktionen werden anschließend direkt auf eine vorbereitete DEAE-®Sepharose (Pharmacia, Schweden), Fast Flow Säule, pH 7,2, 300 ml (5 x 15 cm²) aufgetragen, mit Phosphatpuffer gewaschen und schließlich mit einem 0 bis 0,5 molarem Kochsalzgradienten, pH 7,2 eluiert. Das Protein pGF2 befindet sich in den Fraktionen, in denen die Leitfähigkeit der Lösung 20 bis 30 mS beträgt. Diese werden zusammengefaßt und mit Ammonium-Sulfat (35 % Sättigung) gefällt. Der sich bildende Niederschlag wird nach 16 Stunden abzentrifugiert, mit Wasser gelöst, durch Einstellen des pH-Wertes auf 4,6 erneut gefällt und diesmal bereits nach 2 Stunden mittels Zentrifugation gesammelt. Es enthält das bereits stark angereicherte Protein pGF2. Die Endreinigung erfolgt auf ®Macrobore Nucleosil (Macherey und Nagel, Düren) 20-10 C4, wobei die Säulengröße so bemessen ist, daß 100 mg pGF2 auf 100 ml Säulenvolumen getrennt wird. Das Entwickeln der präparativen HPLC-Säule geschieht ebenfalls mit dem 0,1 Trifluoressigsäure/Acetonitrilsystem. Die Elution des Fusionsproteins erfolgt mit 33 %igem Acetonitril. Trocknung im Vakuum führt zur Festsubstanz.

### Beispiel 2

10 mg Fusionsprotein, das nach Beispiel 1 gewonnen wurde, wird in 0,4 ml Natriumacetatpuffer, pH 5,3 und 0,1 ml DMF gelöst. Dazu werden 140 mg Thr(But)OBut und 50 µl Lysylendopeptidase (2,7 mg/ml; gelöst in Wasser) gegeben. Das Ganze wird bei Raumtemperatur stehengelassen. Mittels HPLC (System wie Beispiel 3) wird die Reaktion analytisch verfolgt.

Zu verschiedenen Zeiten werden Proben genommen, mit dem doppelten Volumen Methanol und dem 10-fachen Volumen an Methyl-t-Butyl-Ether ausgefällt. Der Niederschlag wird in dem 10-fachen Volumen an 0,1 %iger Trifluoressigsäure aufgenommen und in der HPLC analysiert. Nach 40 h sind ca. 55 % Humaninsulin-Ester entstanden. Der Rest ist im wesentlichen Des-B30-Insulin.

### Beispiel 3

Der nach Beispiel 1 gewonnene Niederschlag wird in möglichst wenig 0,1 %iger Trifluoressigsäure aufgenommen und in der HPLC präparativ getrennt. Als stationäre Phase wird eine Bakerbond Wide-Pore^{TM} C₈/5 µm mit den Abmessungen 4,6 x 250 mm eingesetzt. Die mobile Phase setzt sich folgendermaßen zusammen:
A: 0,1 % TFA
B: A/CH₃CN 10/90

Der Fluß beträgt 1,3 ml/min. Der Gradient setzt sich wie folgt zusammen

| t | % B |
|---|---|
| 0 | 25 |
| 20 | 45 |
| 25 | 45 |
| 28 | 25 |
| 35 | 25 |

Des-B³⁰-Insulin eluiert bei 15,14 min, Insulin-Ester bei 20,92 min. Die Fraktionen werden im Vakuum vom Lösungsmittel befreit, der Feststoff mit Acetonitril nachgewaschen und im Vakuum vom Lösungsmittel befreit.

### Beispiel 4

100 mg des Proteins pGF2 werden in 10 ml Tris/HCl-Puffer, pH 8 gelöst und mit 50 mU Lysylendopeptidase inkubiert. Nach 2,5 Stunden wird der Ansatz durch Sauerstellen auf pH 3 mit Trifluoressigsäure gestoppt und die Reaktionsmischung auf Reverse Phase aufgetrennt. Ein scharfer Peak, eluiert mittels 30 %igen Acetonitril markiert das Erscheinen des B³⁰-Desthreonin-Humaninsulins in dem Säulenausfluß. Das vom Lösungsmittel befreite Produkt wiegt 35 mg, entsprechend 95 % der Theorie.

### Beispiel 5

90 mg α-Amylase aus Schweinepankreas werden in 35 ml 0,5 M Kaliumphosphatpuffer, pH 7,8, gelöst und über Nacht gegen den gleichen Puffer dialysiert.

50 g ®Affigel 10 (BioRad 153 - 6046) werden nach Herstellervorschrift gewaschen und mit 0,5 M Kaliumphosphatpuffer (s.o.) äquilibriert. Das so gewonnene Feuchtgel wird mit der Enzymlösung unter leichtem Schütteln bei Raumtemperatur 2 h reagieren gelassen. Danach wird abgesaugt und kurz mit Bindungspuffer gewaschen. Anschließend erfolgt die Inaktivierung des Trägers während einer 1/2 h mit 0,1 M Ethanolamin, pH 8,0, schließlich wird erneut abgesaugt und mehrmals mit 1 M NaCl und Bindungspuffer im Wechsel gewaschen. Man erhält ca. 44 g feuchtes Gel, das bei 4 °C unter 0,02 % Azid in 50 mM Kaliumphosphatpuffer gelagert wird. Es ergibt folgende Bindungsbilanz:
- Ausgang:: 1004 Units = 100 %
- Vereinigte Waschwässer:: 705 Units = 70 %
- Träger:: 181 Units = 18 %
- Testverfahren:: Test-Kit "Testomar" von Behring; Durchführung nach Herstellervorschrift

### Beispiel 6

44 g nach Beispiel 4 fixierte Amylase werden mit 10 mg, nach Beispiel 4 abgebauten pGF2 ohne Reinigung unter Zusatz eines Proteaseinhibitors in 200 ml Wasser verrührt. Nach einer 1/4 Stunde wird abgesaugt und die klare flüssige Phase gefriergetrocknet. Es resultieren 5 mg eines farblosen Produktes, welches in wenig Wasser aufgenommen durch Zn²⁺-Zusatz zu kristallinem Des-B³⁰-Insulin führt.

## Patentansprüche

1. Verfahren zur Herstellung von B³⁰-Desthreonin-Humaninsulin durch enzymatische Spaltung eines Fusionsproteins mit einer Trypsin-ähnlichen Endopeptidase, dadurch gekennzeichnet, daß man ein Fusionsprotein verwendet, daß durch Expression eines Strukturgens in einer Streptomycetenzelle erhalten wird, wobei das Strukturgen aus der Tendamistat-sequenz besteht, auf das die Gensequenz für das Brückenglied mit den Aminosäuren Phe Asn Ala Met Ala Thr Gly Asn Ser Asn Gly Lys folgt und hierauf das Gen für ein menschliches Proinsulinderivat folgt, bei dem die B Kette über ein Brückenglied an die A-Kette gebunden ist, das für Lys oder Lys-Lys codiert.

2. Verfahren nach Anspruch 1, dadurch gekennzeichnet, daß man das Fusionsprotein in einer Konzentration von etwa 0,02-15 Gew.-%, vorzugsweise von etwa 0,5-5 Gew.-%, bezogen auf die gesamte Reaktionsmischung, einsetzt.

3. Verfahren nach Anspruch 1, dadurch gekennzeichnet, daß man als Trypsin-ähnliche Endopeptidase Lysylendopeptidase verwendet.

4. Verfahren nach einem oder mehreren der Ansprüche 1 bis 3, dadurch gekennzeichnet, daß man die Trypsin-ähnliche Endopeptidase in einer Konzentration zwischen etwa ein Fünfzigstel und ein Zehntausendstel, des Gewichts des Fusionproteins der Formel I einsetzt.

5. Verfahren nach einem oder mehreren der Ansprüche 1 bis 4, dadurch gekennzeichnet, daß man die enzymatische Spaltung in wäßriger Lösung, gegebenenfalls in Gegenwart eines Lösungsvermittlers, des pH-Werts zwischen etwa 5 und 11, vorzugsweise zwischen etwa 7 und 9,5, durchführt.

6. Verfahren nach einem oder mehreren der Ansprüche 1 bis 5, dadurch gekennzeichnet, daß man den Reaktionsansatz nach beendeter enzymatischer Spaltung durch Kristallisation aufarbeitet.

## Claims

1. A process for the preparation of B³⁰-dethreonine-human insulin by enzymatic cleavage of a fusion protein with a trypsin-like endopeptidase, wherein a fusion protein obtained by expression of a structural gene in a streptomyces cell is used, where the structural gene comprises the tendamistat sequence followed by the gene sequence for the linker with amino acids Phe Asn Ala Met Ala Thr Gly Asn Ser Asn Gly Lys which is followed by the gene for a human proinsulin derivative in which the B chain is linked via a linker which codes for Lys or Lys-Lys to the A chain.

2. The process as claimed in claim 1, wherein the fusion protein is employed in a concentration of about 0,02-15% by weight, preferably of about 0.5-5% by weight, based on the total reaction mixture.

3. The process as claimed in claim 1, wherein Lysyl endopeptidase is used as trypsin-like endopeptidase.

4. The process as claimed in one or more of claims 1 to 3, wherein the trypsin-like endopeptidase is employed in a concentration between about one fiftieth and one tenthousandths of the weight of the fusion protein of the formula I.

5. The process as claimed in one or more of claims 1 to 4, wherein the enzymatic cleavage is carried out in aqueous solution, where appropriate in the presence of a solubilizer, of pH between about 5 and 11, preferably between about 7 and 9.5.

6. The process as claimed in one or more of claims 1 to 5, wherein the reaction mixture is worked up by crystallization after the enzymatic cleavage is complete.

## Revendications

1. Procédé pour la préparation d'insuline humaine B³⁰-desthréonine par dissociation enzymatique d'une protéine de fusion à l'aide d'une endopeptidase de type trypsine caractérisé en ce qu'on utilise une protéine de fusion qui est obtenue par expression d'un gène de structure dans les cellules de streptomycète, le gène de structure étant constitué d'une séquence tendamistat suivie de la séquence génique correspondant à la chaîne pontante avec les aminoacides Phe Asn Ala Met Ala Thr Gly Asn Ser Asn Gly Lys et laquelle est elle-même suivie du gêne correspondant au dérivé de proinsuline humaine, ce par quoi la chaîne B est lié à la chaîne A par l'intermédiaire d'une chaîne pontante qui code pour lys ou lys-lys.

2. Procédé selon la revendication 1, caractérisé en ce que l'on introduit la protéine de fusion à une concentration d'environ 0,02-15 % en poids, de préférence d'environ 0,5-5 % en poids calculé par rapport au poids total du mélange réactionnel.

3. Procédé selon la revendication 1, caractérisé en ce que l'on utilise en tant qu'endopeptidase de type trypsine la lysylendopeptidase.

4. Procédé selon l'une ou plusieurs des revendications 1 à 3, caractérisé en ce que l'on utilise l'endopeptidase de type trypsine à une concentration comprise entre environ un cinquantième et un dix-millionième du poids de la protéine de fusion de formule I.

5. Procédé selon l'une ou plusieurs des revendications 1 à 4, caractérisé en ce que la dissociation enzymatique est réalisé en solution aqueuse, éventuellement en présence d'un agent de solubilisation, dont le pH est compris entre 5 et 11, de préférence entre 7 et 9,5.

6. Procédé selon l'une ou plusieurs des revendications 1 à 5, caractérisé en ce qu'après achèvement de la dissociation enzymatique, l'on purifie le produit de la réaction par cristallisation.
